# EUROPEAN PATENT APPLICATION

(11) **EP 4 252 719 A1**
(43) Date of publication of application: **04.10.2023**
(21) Application number: 22166334.7
(22) Date of filing: 01.04.2022
(51) Int. Cl.: A61F 2/958, A61M 25/00, A61F 2/954

(54) **CONTROLLED AND PRECISE STENT PLACEMENT AT A CORONARY OSTIUM IN PERCUTANEOUS CORONARY INTERVENTIONS**

(71) Applicant: Erasmus University Rotterdam Medical Center, 3015 GE Rotterdam (NL)
(72) Inventor: WILSCHUT, Jeroen Maarten, 3015 GE Rotterdam (NL)
(74) Representative: V.O.

(57) **Abstract**

A stent delivery system (100) comprises a balloon-inflatable stent (10) with a guide wire (31) for guiding the stent (10) into a target vessel (V1) for treating a stenosis (4) in the target vessel (V1) with the stent (10) to be delivered at a predetermined stent position (Xs). An anchor wire (32) is provided through an anchor port (42) for anchoring the stent (10) by entering into a lumen or second vessel (V2) branching from the target vessel (V1) at a branching position (Xb). The anchor port (42) is freely adjustable along a length (ΔX) of the guide wire (31) for adjusting the anchor position (Xa) relative to the stent position (Xs), or vice versa. A hypotube (41) is connected to the anchor port (42) and configured to control said freely adjustable anchor position (Xa) relative to the stent position (Xs) by pushing and pulling on the hypotube (41).

## Description

### TECHNICAL FIELD AND BACKGROUND

The present invention relates to a system for delivering a stent to a stenosis in target vessel adjacent a branching position where the target vessel branches from a lumen or second vessel (carina or bifurcation). The invention also relates to a kit of parts for building such system, and an anchor port assembly for use in a stent delivery system or kit of parts.

Coronary stenosis is a ubiquitous disease. Since the introduction of percutaneous coronary intervention (PCI) in 1977 by Andreas Grüntzig, both the number of PCI's as well as development of techniques and devices have seen exponential growth. Currently, almost every technical aspect of coronary disease has been addressed including the much awaited successful treatment of chronic total occlusions. The PCI of coronary lesions located at or near a coronary ostium is often pursued and part of mainstream clinical practice, although the procedure can be technically challenging.

The exact positioning of the coronary stent at a coronary ostium, if pursued, is of great importance. Although more difficult than conventional lesions, the operator is limited to the same palette of PCI techniques, including the limitations of 2D angiography and patient induced movement resulting. This can result in excessive radiation, contrast dye use and uncontrolled/undesired coronary stent positioning.

The problem of stent placement can be addressed by the so-called "tail wire" or "Szabo" technique. The tail wire technique uses the most proximal stent strut as an anchor for a second or "anchor" wire to pass through. The anchor wire is positioned in the space in which the stent is not supposed to protrude, usually the aorta or, in case of a bifurcation, the side branch. The anchor prohibits the stent from advancing into the coronary artery, positioning its proximal part exactly at the carina. Although the technique provides more control during stent positioning, which is of great value in stenting at coronary ostia, it also requires stent manipulation and thereby introduces subsequent problems.

In a previous patent publication WO2019/156560A1, by the present inventor, problems of the traditional "tail wire" technique are alleviated by separating the anchoring possibility from the stent. In particular, this patent publication describes a system for delivering a stent to a predetermined stent position in a target vessel. The stent is wrapped around an inflatable balloon with a guide channel. A guide wire for entering into the target vessel passes through the guide channel for guiding the stent into the target vessel. A part of the inflatable balloon is wrapped by an elastic sleeve. The sleeve also provides an anchor port separate from the stent struts for guiding an anchor wire, e.g. into a branching vessel. Additionally, the sleeve is configured to at least partly prevent or delay the inflating of the inflatable balloon at the wrapped part compared to another part of the inflatable balloon opposite the stent. The anchor port can be connected to an anchor port line configured to remotely control the exit direction of the anchor port.

As further background, US 2017/0274179 A1 describes a hypotube construction. As explained in this prior art, a hypotube is typically a long metal tube with micro-engineered features along its length. A hypotube can be used as a component of minimally-invasive catheters, in conjunction with balloons and stents to open up clogged arteries. Typically, the balloon portion of the catheter can be attached to a head of the hypotube. A hypotube may have a reinforcement which may give its liners more support and integrity as the catheters navigate the vasculature to a treatment location. In some instances, a polymer jacket is distributed on an outer diameter of the hypotube to provide a seal and also to minimize any surface roughness imparted by the laser cutting of the hypotube while still providing flexibility.

There is yet a need for further improvements that can alleviate one or more problems of known stent delivery systems while maintaining at least some of their advantages. In particular, the inventor has recognized a need to more freely determine an exact position of the stent placement relative to a branching vessel structure while maintaining necessary precision and control over the stent deployment.

### SUMMARY

Aspects of the present invention relate to a system or kit of parts for delivering a stent. In particular the system is configured for delivery of the stent to a predetermined stent position in a target vessel adjacent a branching position where the target vessel branches from a lumen or second vessel. The stent delivery system comprises a balloon-inflatable stent with a guide wire for entering into the target vessel. By also providing an anchor or tail wire, which can enter into an branching vessel, the position of the stent can be anchored relative to the branching position. In particular, the anchor wire can pass through an anchor port to fix the position accordingly.

Contrary to previous solutions, the position of the anchor port, as described herein, is freely adjustable along a length of the guide wire relative to the stent position. Similarly, the stent position can also be adjusted with respect to the anchor position (which is determined by the anchor wire relative to the branching position). As will be appreciated, this configuration can provide improved control over the exact stent position while maintaining precision provided by the anchoring relative to the branching position. For example, this may be compared to other solutions, wherein the anchor port is formed by a stent strut (as in the traditional Szabo technique), or fixated relative to the stent such as disclosed in the inventor's above mentioned previous patent publication by connection to a sleeve that is immobile by being wrapped around the balloon and therefore not freely adjustable. Furthermore, as described herein, a hypotube is connected to the anchor port for controlling said freely adjustable position by pushing and pulling on the hypotube. This is different from the inventor's above mentioned previous patent publication where the anchor port is optionally connected to an anchor port line but only for adjusting its orientation, not suitable to change relative position with respect to the balloon - at least not by pushing on the wire. Together, the distinguishing features allow more freedom to adjust the exact position relative to the balloon/stent where the main wire and anchor wire split up into the respective vessels. Further synergetic aspects include providing the anchor port with a radiopaque material that allows the device to be precisely placed. Further aspects may also include the ability to form the anchor port as a widening tubular member which can slide over a proximal part of the balloon and prevent or delay inflation thereof.

Other or further aspect relate to an anchoring assembly for supplementing a balloon-inflatable stent delivery system. The assembly can, e.g., act as a visual and mechanical aid to position and/or anchor a coronary stent exactly at a coronary ostium, e.g. defined by the so-called "carina". Also other types of stents may benefit from the assembly. For example, while the precision of the exact positioning in coronary vessels is most challenging, similar techniques can also be applied in other (larger) vessels, such as bifurcations at the renal vessels (arteries and veins), leg vessels, brain vessels, et cetera. As described herein, the device preferably consists of a hypotube which is connected to a ring or tubular extension which may contain a radiopaque material, to define the exact location of an ostium, enabling the operator to aim the desired stent position precisely, in particular by using two separate guidewires in combination, one positioned in the target vessel, the other position in the adjacent structure (e.g. branching vessel). For example, after positioning of both guidewires the ostium can be marked by carefully pushing the tubular extension until resistance is met, mechanically and subsequently visually identifying the carina.

### BRIEF DESCRIPTION OF DRAWINGS

These and other features, aspects, and advantages of the apparatus, systems and methods of the present invention will become better understood from the following description, appended claims, and accompanying drawing wherein:
FIGs 1A and 1B illustrate delivery and deployment of a stent to treat a stenosis in a target vessel near a bifurcation with a second vessel;
FIG 2A illustrates details of an anchoring assembly for use in a stent delivery system; and
FIGs 2B - 2D illustrate different anchoring assemblies.

### DESCRIPTION OF EMBODIMENTS

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise.

The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

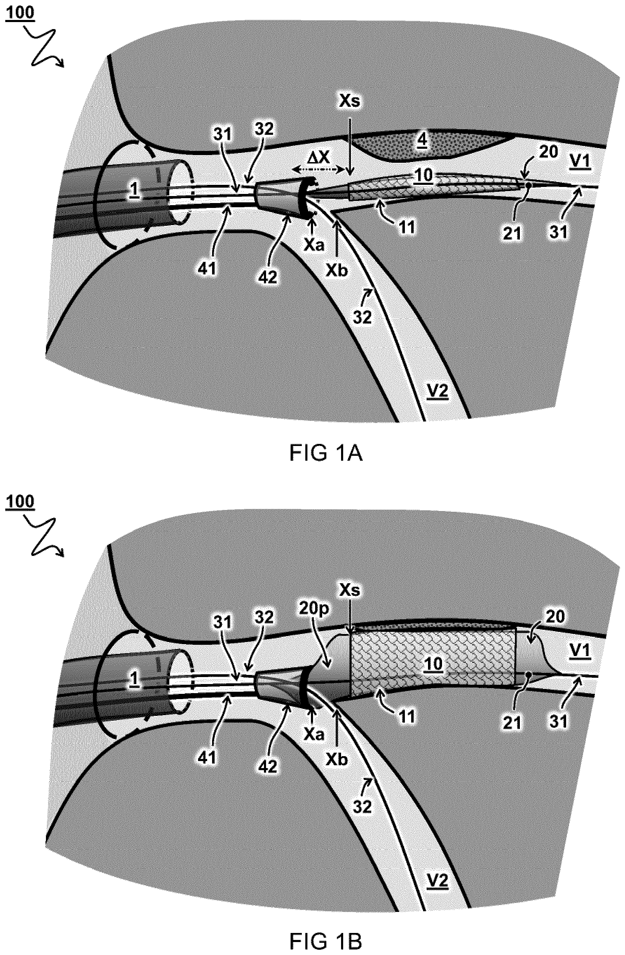

FIGs 1A and 1B illustrates delivery and deployment of a balloon-inflatable stent 10 to treat a stenosis 4 in a target vessel V1 near a bifurcation with a second vessel V2.

In some embodiments, the system comprises an inflatable balloon 20. In one embodiment, the stent 10 comprises an extendable frame 11 wrapped around the inflatable balloon 20. In another or further embodiment, the system comprises a guide wire 31 for entering into the target vessel V1. For example, the guide wire 31 may pass through a guide channel 21 inside the stent or balloon for guiding the inflatable balloon 20 with the stent 10 (while crimped) along or with the guide wire 31 into the target vessel V1.

In some embodiments, the system comprises an anchor wire 32 for determining an anchor position Xa relative to a branching position Xb where the target vessel V1 branches from a lumen or second vessel V2. In one embodiment, the system comprises an anchor port 42 for guiding the anchor wire 32 there through and determining the anchor position Xa relative to the stent position Xs. In one preferred embodiment, the anchor position Xa of the anchor port 42 is freely adjustable along a length ΔX of the guide wire 31 relative to the stent position Xs of the stent 10. In another or further preferred embodiment, a hypotube 41 is connected to the anchor port 42 for controlling said freely adjustable anchor position Xa relative to the stent position Xs, e.g. by pushing and pulling on the hypotube 41. For example, the guide wire 31 and anchor wire 32 may be first positioned into the respective vessels V1,V2; after which the anchor port 42 is guided outside the patient over the two wires and then advanced to the carina.

In some embodiments, the anchor port 42 is formed by a ringshaped and/or tubular member connected to the hypotube 41. In one embodiment, the guide wire 31 passes through the anchor port 42. For example, a proximal part of the guide wire 31 can be inside a second hypotube connected to the balloon-inflatable stent. In another or further embodiment, each of the guide wire 31 and the anchor wire 32 pass through the same opening of the anchor port 42, e.g. through the ring or tube forming said anchor port. For example, this provides an easy construction with a single opening to guide both wires (and also the stent). Alternatively there could be separate openings for the respective wires.

In some embodiments, the anchor port 42 is configured to fit at least part of the inflatable balloon 20 inside. For example, part of the inflatable balloon 20 (before deployment) may fit inside the anchor port 42, e.g. up to the extendable frame 11, or further. By fitting part of the balloon 20 inside the anchor port 42, while the balloon is being inflated, the anchor port 42 can at least partially prevent and/or delay inflation of said part of the balloon. This may be advantageous in further controlling the inflation process, e.g. prevent the stent frame from shifting during inflation.

In some embodiments, the crimped stent 10, i.e. undeployed stent frame 11 around the deflated balloon, fits completely inside and/or can be guided through the anchor port 42. This may be advantageous, e.g. if the stent 10 is sent along the guide wire 31 after the position of the anchor port 42 is established. For example, FIG 1B illustrates a deployed stent wherein a proximal side 20p of the inflated balloon is partially inflated inside the tubular extension forming the anchor port 42. By preventing or delaying the inflation, it may be prevented, e.g., that the stent frame moves away from the intended position during inflation.

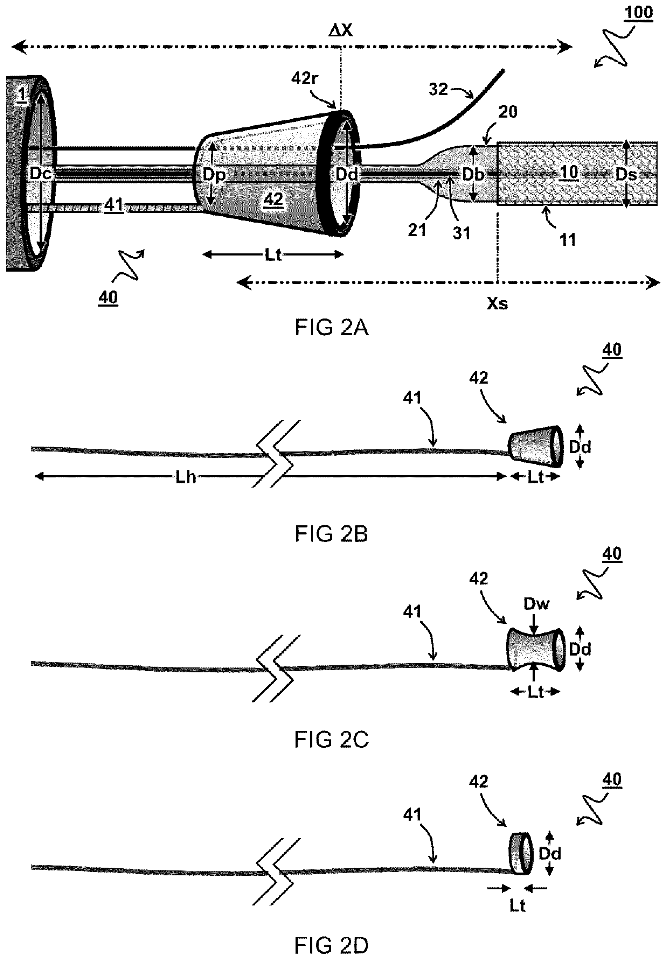

FIG 2A illustrates details of an anchoring assembly 40 for use in a stent delivery system 100, e.g. as described herein. For example, anchoring assembly 40 can be a stand-alone device used to supplement an existing stent delivery system.

In some embodiments, the anchor port 42 (e.g. tubular member as shown or other tube or ring-shape) has a (minimum) inner diameter Dp that is larger than a cumulative outer diameter of the guide wire 31 and the anchor wire 32, e.g. by at least a factor 1.1, 1.5, 2, or more. For example, the minimum inner diameter Dp is more than twice the outer diameter of each of the guide wire 31 and anchor wire 32. This allows each of the guide wire 31 and anchor wire 32 to freely slide through the anchor port 42 and/or allows the anchor port 42 to be freely adjustable along said wires. Typically the anchor port 42 is at least larger in diameter than the hypotube 41 used to control its position. In one embodiment, the anchor port 42 has an outer diameter larger than an outer diameter of the hypotube 41, e.g. by at least a factor two, four, six, eight, ten, or more.

In some embodiments, the anchor port 42 is formed by a tubular member shaped to slide over a proximal region of the inflatable balloon 20 for at least partially preventing and/or delaying inflation at said proximal region. In one embodiment, the anchor port 42 has an inner diameter Dd, at least at a distal side facing a proximal side of the inflatable balloon 20, that is larger than an outer diameter Db of the inflatable balloon 20 (before inflation). This allows at least part of the inflatable balloon 20 to be fitted inside the anchor port 42, as noted above.

In some embodiments, the anchor port 42 has a minimum inner diameter Dp that is larger than an outer diameter Ds of the (crimped) stent 10 before inflation of the balloon 20. This allows to fit the crimped stent 10 inside and pass through the anchor port 42. In other or further embodiments, the anchor port 42 has a minimum inner diameter Dp that is (slightly) larger than a cumulative outer diameter of the anchor wire 32 and the (crimped) stent 10 before inflation of the balloon 20, e.g. larger by a factor between 1.01 and 1.5, preferably between 1.05 (5% larger) and 1.3 (30% larger). This allows to fit the crimped stent 10 inside and pass through the anchor port 42 also when the anchor wire 32 is already passed through the anchor port 42.

In a typical balloon-inflatable stent, before deployment, the outer diameter Ds of the (crimped) stent is typically between 3 and 12 mm, more commonly between 5 and 10 mm. The minimum inner diameter Dp of the anchor port 42 is preferably slightly larger, e.g. at least one millimeter larger than the outer diameter Ds of a crimped stent. For example, a thickness of the anchor wire 32 is typically less than one millimeter. Accordingly, in one embodiment, the minimum inner diameter Dp of the anchor port 42 is between 4 and 13 mm, or between 6 and 11 mm, or more, depending on the stent. When deployed, the diameter may increase, e.g. by a factor between 1.5 and 2.5, or more. Typical lengths of the stent (frame) may vary, e.g. between 8 and 40 mm, more commonly between 10 and 30 mm, or between 12 and 20.

In some embodiments, the system 100 comprises a (coronary) guiding catheter 1 having an inner diameter Dc configured to fit a maximum outer diameter of the anchor port 42 inside for guiding the anchor port 42 through the guiding catheter 1. For example, the maximum outer diameter of the anchor port 42 is slightly larger than its maximum inner diameter Dd (depending on a thickness of the tube wall or ring). In other or further embodiments, the hypotube 41 is configured to push the anchor port 42 through a guiding catheter 1, and beyond a distal end of the guiding catheter 1 along the guide wire 31 and/or anchor wire 32.

In some embodiments, the anchor port 42 is shaped as a tubular member having a length Lt that is larger than its (minimum and/or maximum) inner diameter Dp, Dd. This may allow further control, e.g. prevent rotation of the tubular axis relative to the wire. In a typical balloon-inflatable stent, the inflatable balloon, or at least the part that expands in diameter, extends beyond the edge of the stent frame, e.g. by at least 2 mm, more commonly at least 4 mm, e.g. between 5 and 10 mm, or more. In one embodiment, the length Lt of the anchor port 42 is the same or similar to the length of said (inflatable) part of the balloon extending beyond the stent frame, e.g. the same within a factor 1.2, or a factor 1.5, up to a factor two, or more. Accordingly, in one embodiment, the length Lt of the of the anchor port 42 is between 2 and 20 mm, more commonly between 4 and 15 mm, or between 6 and 10 mm, depending on the stent. Alternatively, e.g. as shown in FIG 2D, the tubular member could also be embodied as a relatively short tube, e.g. ring.

In some embodiments, e.g. as shown in FIG 2B, the anchor port 42 is formed by an extended tubular member having a proximal end, where the anchor port 42 is connected to the hypotube 41, and a distal end, opposite the proximal end, wherein the extended tubular member has an (inner) diameter Dd that increases towards said distal end. For example, the increasing diameter may allow a relatively large opening angle between the guide wire 31 and anchor wire 32 exiting the distal end of the tubular member while still substantially preventing or delaying inflation of part of the balloon 20. For example, the tubular member has a frusto-conical hollow shape. Also other diverging shapes could be used. In other or further embodiments, e.g. as shown in FIG 2C, wherein the anchor port 42 is formed by an extended tubular member having an (inner) diameter Dd that increases towards both the distal and proximal ends. For example, the tubular member has a relatively low waist diameter Dw relative to the diameter Dd,Dp at the distal and/or proximal ends, e.g. lower by at least 10%, 20%, up to 50%, or more. In other or further embodiments (not shown), the tubular member could be a straight tube, e.g. with relatively large diameter to allow the large opening angle.

In some embodiments, the anchor port 42 comprises a rigid material, e.g. compared to a material of the balloon 20. For example, the balloon is made of rubber or nylon. For example, the material of the anchor port 42 has Young's modulus more than 0.1 GPa, typically more than 1 GPa. In one embodiment, the anchor port 42 comprises a tubular member having a (relatively rigid) mid-section and at least one end section having higher flexibility than the mid-section. For example, by providing one or both end sections with more flexibility, this may facilitate larger angles between the guide wire 31 and anchor wire 32 guided there through while maintaining prevention or delay of inflation of balloon.

In some embodiments, the anchor port 42 comprises a radiopaque material 42r. The skilled person will understand that a radiopaque material has a relatively high absorbance for X-rays, at least to such a degree that it is normally visible and can be distinguished (e.g. from biological tissue background) in a radiological image of a body. In contrast, a radiolucent or radiotransparent material is essentially transparent to X-rays so that it is normally invisible or barely visible in a radiological image, e.g. having similar X-ray characteristics as tissue background. So by providing the anchor port 42 with a radiopaque material 42r, an operator of the system can easily discern a position of the anchor port 42 inside the body using radiological / X-ray imaging. Typically, at least part of the stent, e.g. outer edges, are also provided with a radiopaque material. This allows to determine in a radiographic image where the stent is located. For example, the radiopaque material 42r of the anchor port 42 can be the same or similar as the radiopaque material provided on the stent. Providing both the anchor port 42 and stent 10 with respective radiopaque material, allows to determine in a radiographic image where the anchor port 42 is located relative to the stent, which may help further control its deployment.

In some embodiments, the anchor port 42 has a tubular shape and comprises a radiopaque material 42r disposed (at least or exclusively) at a distal edge of the tubular shape (opposite the hypotube 41), e.g. forming a radiopaque ring at said distal edge. In this way the exact position of the distal end can be more precisely distinguished, which is the most relevant as it forms the exit port for the different wires and determines a position where inflation of the balloon may be prevented. For example, the rest of the tubular shape, e.g. proximal region, is formed by a radiolucent or radiotransparent material, e.g. at least more transparent to X-rays than the radiopaque material 42r.

As described herein, the hypotube 41 is preferably configured to allow various forms of control over positioning of the anchor port 42, e.g. allowing the anchor port 42 to be pushed towards the stent/balloon, most preferably also allowing control over a rotation of the anchor port 42. For example, the hypotube 41 is attached to a proximal edge of the anchor port 42. Hypotubes are typically manufactured having a diameter between 0.2 - 5 mm. In some embodiments, the system comprises multiple hypotubes. In one embodiment, the anchor port 42 is connected to a first hypotube 41 and the balloon-inflatable stent 42 is connected to a, separate, second hypotube. In principle, the second hypotube can be the same or similar as the first hypotube. Typically, the second hypotube connected to the stent may be different, e.g. having a larger diameter to accommodate the balloon inflation channel inside. Also the guide wire 31 could be accommodated inside the second hypotube leading to the balloon-inflatable stent.

For the present purposes, the hypotube 41 connected to the anchor port 42 preferably has a relatively low (inner and outer) diameter, e.g. less than 2 mm, less than 1 mm, less than 0.5 mm, or even less than 0.3 mm. Typically the hypotube 41 is hollow, although the tube may also be filled if it is used only to manipulate the anchor port 42. The hypotube has specific advantages of control at minimal thickness, although in principle it could also be substituted for tube and/or wire-like structures, e.g. nitinol, that allow similar manipulation such as pushing and pulling on the anchor port 42. In one embodiment, the hollow hypotube 41 is additionally used as a guide, e.g. used to guide the anchor wire 32 inside. For example, a proximal end of the hollow hypotube 41 may end inside or close before (proximal) the anchor port 42 so the anchor wire 32 can exit through the anchor port 42.

In some embodiments, the hypotube 41 is formed by long tube, e.g. formed of metal or other material, typically having micro-engineered features along its length. For example, the micro-engineered features may include one or more of a continuous or interrupted spiral cut pattern, radial cut pattern, cut holes, et cetera. To facilitate deployment and/or prevent damage, the hypotube 41 may be coated, e.g. with Teflon. Typically, the hypotube 41 has a similar length as the wires 31,32 and/or channel leading to the inflatable balloon. For example, the length Lh of the hypotube 41 is at least half a meter, more commonly at least one meter, e.g. between 1.2 and 2.5 meter, or more. Each of the hypotube 41 and anchor port 42 is typically of a medical grade material.

In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise. Where one claim refers to another claim, this may indicate synergetic advantage achieved by the combination of their respective features. But the mere fact that certain measures are recited in mutually different claims does not indicate that a combination of these measures cannot also be used to advantage. The present embodiments may thus include all working combinations of the claims wherein each claim can in principle refer to any preceding claim unless clearly excluded by context.

## Claims

1. A stent delivery system (100) comprising
a balloon-inflatable stent (10) comprising a stent frame (11) wrapped around an inflatable balloon (20) and having a guide wire (31) for guiding the stent (10) into a target vessel (VI) for treating a stenosis (4) in the target vessel (VI) with the stent (10) to be delivered at a predetermined stent position (Xs);
an anchor wire (32) for anchoring the stent (10) by entering into a lumen or second vessel (V2) branching from the target vessel (VI) at a branching position (Xb);
an anchor port (42) for guiding the anchor wire (32) there through and determining an anchor position (Xa) relative to the stent position (Xs),
wherein the anchor position (Xa) of the anchor port (42) is freely adjustable along a length (ΔX) of the guide wire (31) for adjusting the anchor position (Xa) relative to the stent position (Xs), or vice versa; and
a hypotube (41) is connected to the anchor port (42) and configured to control said freely adjustable anchor position (Xa) relative to the stent position (Xs) by pushing and pulling on the hypotube (41).

2. The system according to claim 1, wherein the anchor port (42) is formed by a ring or tubular-shaped member connected at a distal end of the hypotube (41) and having an inner diameter (Dp,Dd) that is larger than an outer diameter of the hypotube (41) by at least a factor two.

3. The system according to any of the preceding claims, wherein the anchor port (42) is formed by a tubular member having a maximum inner diameter (Dd), at least at a distal side facing a proximal side of the inflatable balloon (20), that is larger than an outer diameter (Db) of the inflatable balloon (20) before inflation, wherein the tubular member is shaped to slide over a proximal region of the inflatable balloon (20) for at least partially preventing and/or delaying inflation at said proximal region, wherein an extent by which said tubular member slides over said proximal region is controllable via the hypotube (41).

4. The system according to any of the preceding claims, wherein the anchor port (42) has a length (Lt) that is the same, or similar within a factor two, as a length of an inflatable part of the balloon extending beyond the stent frame (11).

5. The system according to any of the preceding claims, wherein the anchor port (42) is formed by an extended tubular shape having an inner diameter that increases along its length (Lt) from a relatively narrow mid-section diameter (Dw) towards one or both ends of the tubular shape, ending in a relatively wide distal diameter (Dd) and/or relatively wide proximal diameter (Dp).

6. The system according to any of the preceding claims, wherein the anchor port (42) is formed by a frusto-conical hollow shape member having a length (Lt) that is larger than its maximum inner diameter (Dd).

7. The system according to any of the preceding claims, wherein the anchor port (42) comprises a material that is more rigid than a material forming the balloon (20).

8. The system according to any of the preceding claims, wherein the anchor port (42) comprises a tubular shape having a relatively rigid mid-section and at least one end section having higher flexibility than the mid-section.

9. The system according to any of the preceding claims, wherein each of the guide wire (31) and the anchor wire (32) passes through the same opening formed by the anchor port (42)

10. The system according to any of the preceding claims, wherein the anchor port (42) has a minimum inner diameter (Dp) that is larger than a cumulative outer diameter of the anchor wire (32) and the crimped stent (10) before inflation of the balloon (20).

11. The system according to any of the preceding claims, wherein the system (100) comprises a guiding catheter (1) having an inner diameter (Dc) configured to fit a maximum outer diameter of the anchor port (42) inside for guiding the anchor port (42) through the guiding catheter (1), wherein the hypotube (41) is configured to push the anchor port (42) through the guiding catheter (1), and beyond a distal end of the guiding catheter (1) along the guide wire (31) and anchor wire (32).

12. The system according to any of the preceding claims, wherein the anchor port (42) has a tubular shape and comprises a radiopaque material (42r) disposed exclusively at a distal edge of the tubular shape to form a radiopaque ring at said distal edge.

13. The system according to any of the preceding claims, wherein the hypotube (41) is formed by tube of metal, or other rigid material, having micro-engineered features along its length for providing flexibility to the tube.

14. A kit of parts for assembling the stent delivery system (100) according to any of the preceding claims.

15. An anchor port assembly (40) for use in the stent delivery system (100) or kit of parts according to any of the preceding claims, the assembly (40) comprising
a hypotube (41) having an outer diameter less than two millimeter, and a length of at least one meter; and
an anchor port (42) formed by a tubular shape connected to a distal end of the hypotube (41) and having a minimum inner diameter (Dp) between four and thirteen millimeter, wherein a distal edge of the tubular shape is formed by a ring of radiopaque material (42r).
